(19) Europäisches Patentamt
European Patent Office
Office européen des brevets

(11) Veröffentlichungsnummer: **0 339 556 B1**

# (12) EUROPÄISCHE PATENTSCHRIFT

(45) Veröffentlichungstag der Patentschrift: **05.07.95**

(21) Anmeldenummer: **89107415.5**

(22) Anmeldetag: **25.04.89**

Die Akte enthält technische Angaben, die nach dem Eingang der Anmeldung eingereicht wurden und die nicht in dieser Patentschrift enthalten sind.

(51) Int. Cl.[6]: **C07D 487/04**, C07D 487/14, C07D 239/92, C07D 239/70, C09B 57/00, C09B 67/22, //(C07D487/04,239:00,237:00), (C07D487/14,239:00,237:00, 209:00)

## (54) 9-Oxo-1,9a-10-triaza-9-hydroanthracen-Verbindungen, Verfahren zu ihrer Herstellung und ihre Verwendung als Farbmittel.

(30) Priorität: **29.04.88 DE 3814552**

(43) Veröffentlichungstag der Anmeldung:
**02.11.89 Patentblatt 89/44**

(45) Bekanntmachung des Hinweises auf die Patenterteilung:
**05.07.95 Patentblatt 95/27**

(84) Benannte Vertragsstaaten:
**CH DE FR GB IT LI**

(56) Entgegenhaltungen:
**DE-A- 3 623 335**
**US-A- 4 250 177**

**DYES AND PIGMENTS, Band 7, Nr. 1, Januar 1986, Seiten 23-32, Elsevier Applied Science Publishers Ltd, GB; A. MARRACCINI et al.: "Novel colorants based on the 9-oxo-1, 9a, 10-triaza-9-hydroanthracene chromophoric system"**

(73) Patentinhaber: **HOECHST AKTIENGESELLSCHAFT**
**D-65926 Frankfurt (DE)**

(72) Erfinder: **Dietz, Erwin, Dr.**
**St.-Matthäus-Strasse 7**
**D-6233 Kelkheim(Taunus) (DE)**
Erfinder: **Prokschy, Frank, Dr.**
**Drosselweg 3**
**D-6230 Frankfurt am Main 80 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Gegenstand der Erfindung sind neue Derivate des 9-Oxo-1,9a,10-triaza-9-hydro-anthracens, die gelb bis violett gefärbte Verbindungen hoher Farbstärke darstellen, Verfahren zu ihrer Herstellung und ihre Verwendung zum Einfärben synthetischer und natürlicher Materialien, beispielsweise zum Pigmentieren von Lacksystemen und Kunststoffen.

Farbige Derivate, die sich vom 9-Oxo-1,9a,10-triaza-9-hydro-anthracen der Formel A

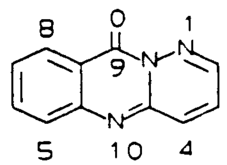

(A)

ableiten, wurden erstmals in DYES AND PIGMENTS 7, 23 (1986) beschrieben:
Man erhält durch Umsetzung von Anthranilsäure B mit Acetanhydrid und Reaktion des dadurch erhaltenen Produkts mit Hydrazin das 3-Amino-2-methyl-4-oxochinazolin C:

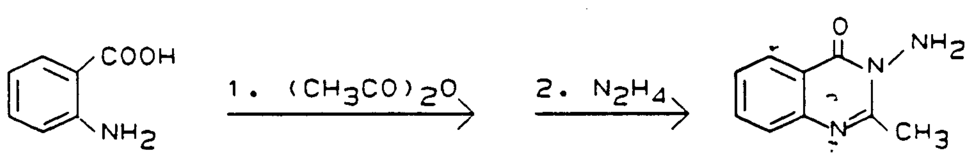


B

C

Durch Umsetzung der Verbindung C mit Acenaphthenchinon, Phenanthrenchinon bzw. Isatin erhält man die Polycyclen D, E und F, die gelb bis orange gefärbte Verbindungen darstellen:

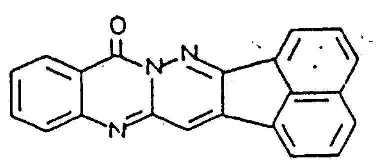

D

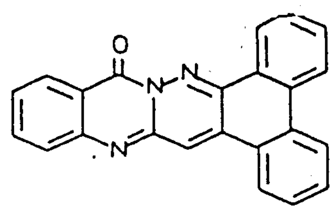

E

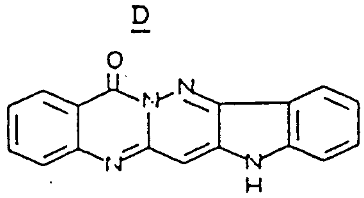

F

+

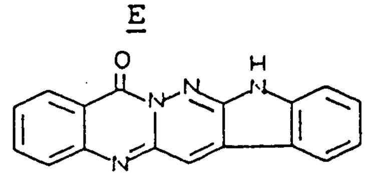

(Isomeres zu F)

Die vorliegende Erfindung betrifft neue Derivate des 9-Oxo-1,9a,10-triaza-9-hydro-anthracens der allgemeinen Formel I

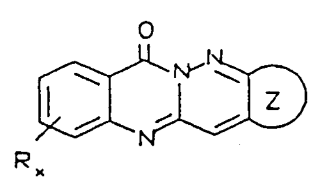

(I) ,

in welcher (Formel I) R ein Halogenatom, vorzugsweise ein Chlor- oder Bromatom, eine der Gruppen Alkyl-$C_1$-$C_4$, Alkoxy-$C_1$-$C_4$ oder Trihalogenmethyl, vorzugsweise Trifluormethyl oder Trichlormethyl, -CO-$NH_2$, CO-NH-(alkyl-$C_1$-$C_4$) oder -CO-N-(alkyl-$C_1$-$C_4$)$_2$ oder einen über das Brückenglied -S-, -SO- oder -$SO_2$-gebundenen Rest, beispielsweise eine der Gruppen Phenyl, Alkyl-$C_1$-$C_4$, -$NH_2$, -NH-(alkyl-$C_1$-$C_4$), -N-(alkyl-$C_1$-$C_4$)$_2$ oder -alkylen-$C_1$-$C_4$-$OSO_3H$, oder einen ankondensierten 4- bis 7-gliedrigen Iso- oder Heterocyclus, beispielsweise aus der Reihe Cyclobutan, Cyclopentan, Thiophen, Pyrrol, Furan, Cyclohexan, Pyridin, Pyrimidin und Cycloheptan, bedeutet, welcher selbst Bestandteil eines polycyclischen Systems, beispielsweise aus der Reihe Naphthalin, Acenaphthen, Phenanthren und Indol sein kann, x eine ganze Zahl von 1 bis 4 bedeutet, wobei für x > 1 R gleich oder verschieden sein kann, und Z einen ankondensierten 4- bis 7-gliedrigen Iso- oder Heterocyclus aus der Reihe Cyclobutan, Cyclopentan, Thiophen, Pyrrol, Furan, Cyclohexan, Pyridin, Pyrimidin und Cycloheptan, darstellt, welcher selbst Bestandteil eines polycyclischen Systems aus der Reihe Naphthalin, Acenaphthen, Phenanthren und Indol, sein kann, wobei jeweils der genannte 4- bis 7-gliedrige Iso- oder Heterocyclus und jeweils das genannte polycyclische System durch Halogenatome, eine der Gruppen Alkyl-$C_1$-$C_4$, Alkoxy-$C_1$-$C_4$, Trihalogenmethyl, -CO-$NH_2$, -CO-NH-(alkyl-$C_1$-$C_4$) oder -CO-N-(alkyl-$C_1$-$C_4$)$_2$ oder über das Brückenglied -S-, -SO- oder -$SO_2$- gebundene Phenyl-, Alkyl-$C_1$-$C_4$, -$NH_2$-, -NH-(alkyl-$C_1$-$C_4$)-, -N-(alkyl-$C_1$-$C_4$)$_2$- oder -alkylen-$C_1$-$C_4$-$OSO_3H$-Gruppen substituiert sein kann.

Die vorliegende Erfindung betrifft ferner ein Verfahren zur Herstellung der neuen Verbindungen der genannten allgemeinen Formel I, indem man Anthranilsäurederivate der allgemeinen Formel II

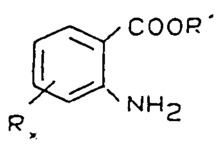

(II)

in welcher R' ein Wasserstoffatom oder eine Alkyl$C_1$-$C_4$ oder Arylgruppe, beispielsweise eine Phenyl- oder Toluylgruppe, darstellt, R und x die weiter obengenannten Bedeutungen haben, zunächst mit 1 bis etwa 50 Mol, vorzugsweise etwa 4 bis etwa 15 Mol Acetanhydrid bei Temperaturen von etwa 50 bis etwa 180°C, ggf. bei erhöhtem oder vermindertem Druck, vorzugsweise bei der Siedetemperatur des Acetanhydrids bei Normalbedingungen, wobei das Acetanhydrid gleichzeitig als Reaktionsmedium dient, dann mit 1 bis etwa 10 Mol, vorzugsweise etwa 2 bis etwa 6 Mol Hydrazin bei Temperaturen von etwa 0 bis etwa 120°C, ggf. bei erhöhtem oder vermindertem Druck, vorzugsweise von etwa 15 bis etwa 70°C bei Normaldruck umsetzt, anschließend das erhaltene Reaktionsprodukt der allgemeinen Formel III

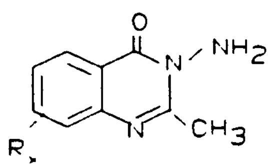

(III)

in welcher R und x die vorstehend genannten Bedeutungen haben, mit 1 bis etwa 2 Mol, vorzugsweise etwa 1,1 bis etwa 1,2 Mol, Diketon der allgemeinen Formel IV

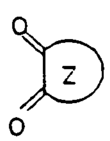

(IV)

in welcher Z die weiter oben gennante Bedeutung hat, bei Temperaturen von etwa 140 bis etwa 240°C, vorzugsweise von etwa 160 bis etwa 180°C, in einem den Reaktionsteilnehmern gegenüber inerten organischen Lösungsmittel, ggf. bei erhöhtem oder vermindertem Druck, unter gleichzeitiger Entfernung des bei der Reaktion entstehenden Wassers umsetzt.

Als bei der letzten Stufe eingesetzte, gegenüber den Reaktionspartnern inerte organische Lösemittel können beispielsweise ggfs. substituierte aliphatische oder aromatische Kohlenwasserstoffe, Ether, Amide oder Gemische davon, bevorzugt o-Dichlorbenzol, Diisopropylnaphthalin (Isomerengemisch), 1-Chlornaphthalin oder eine Mischung aus 76,5 % Diphenylether und 23,5 % Diphenyl (®Dowtherm A) verwendet werden.

Die in zweiter Stufe vorzunehmende Umsetzung mit Hydrazin kann auch in alkoholischem Medium, beispielsweise in Methanol oder Ethanol, durchgeführt werden.

Bei der in erster Stufe erfolgenden Umsetzung der Anthranilsäurederivate der allgemeinen Formel II mit Acetanhydrid reicht an sich ein Molverhältnis von 1 : 1 aus. Im Hinblick darauf aber, daß überschüssiges Acetanhydrid zweckmäßigerweise als Reaktionsmedium dient, empfiehlt sich die Anwendung des Acetanhydrids in einem mehr oder weniger großen molaren Überschuß, beispielsweise in einem Molverhältnis von 1 : 1 bis zu 1 : etwa 50.

Die verfahrensgemäß erhaltenen Verbindungen der allgemeinen Formel I stellen, je nach Art der Substitution, gelb bis violett gefärbte, zum größten Teil schwerlösliche Verbindungen dar, die hohe Farbstärke besitzen und sich zum Einfärben synthetischer und natürlicher Materialien eignen, beispielsweise zum Pigmentieren von Lacksystemen und Kunststoffen.

Die Verbindungen der Formel I fallen bei der Synthese in einem hochsiedenden Lösemittel hochkristallin an und sind in dieser Form zum Anfärben verschiedener Materialien in der Regel nicht geeignet. Sie müssen erst durch geeignete Maßnahmen in eine feiner verteilte Form überführt werden. Solche Maßnahmen können beispielsweise Mahlungen sein, oder auch Umfällungen, in dem man die Verbindung in einem geeigneten Medium löst und wieder ausfällt. Dafür eignen sich neben Polyphosphorsäure und Trifluoressigsäure besonders 96 bis 100 %ige Schwefelsäure. Die darin erhaltene Lösung wird anschließend durch Eingießen in Wasser hydrolysiert. Man erhält sehr fein verteilte Teilchen, die beim Pigmentieren von beispielsweise Acryl-Melamin-Lacksystem oder Polyolefinen eine hohe Farbstärke besitzen. Bei dieser Nachbehandlung der erhaltenen Verbindungen der allgemeinen Formel I zeigt es sich nach Anfertigung von Röntgenbeugungsdiagrammen, daß eine Verbindung in verschiedenen Kristallmodifikationen vorliegen kann.

Je nach Verwendungszweck lassen sich die nach der Hydrolyse angefallenen Pigmente in eine deckendere Form bringen, indem man sie gleich nach der Hydrolyse in dem entstandenen Medium (z.B. in mit Wasser verdünntem schwefelsauren Medium) auf Temperaturen von 60 bis 100 °C erhitzt. Man kann aber das feinverteilte Pigment auch zuerst isolieren und dann in wäßrigem Medium unter Zusatz eines organischen Lösemittels, wie z.B. eines Alkohols, beispielsweise von Ethanol oder Isobutanol, oder einer aromatischen Verbindung, wie beispielsweise Toluol, oder eines Carbonsäureamids, wie beispielsweise Dimethylformamid, gegebenenfalls unter Druck, auf Temperaturen von etwa 80 bis etwa 150 °C erhitzen.

Ferner können die erfindungsgemäßen Pigmente der allgemeinen Formel I auch durch Mahlungen in eine für die Anwendung geeignete Form überführt werden. Diese Mahlungen können in Gegenwart von Wasser und/oder eines wie vorstehend beschriebenen organischen Lösemittels vorgenommen werden. Die Mahlungen können auch in Gegenwart eines anorganischen Salzes als Mahlhilfsmittel, wie beispielsweise Natriumsulfat oder Natriumchlorid, oder aber auch ohne Mahlhilfsmittel vorgenommen werden.

Bei diesen Operationen können durch einen Zusatz einer oberflächenaktiven Verbindung die anwendungstechnischen Eigenschaften dieser neuen Farbmittel deutlich verbessert werden.

Die Methoden zur Erzielung bestimmter Kristalleigenschaften, wie beispielsweise spezielle Oberfläche, Korngrößenverteilung, Teilchenform hängen bei den erfindungsgemäßen Farbmitteln sehr von der Art und der Anzahl der vorhandenen Substituenten ab.

Weiterhin kann es, um bestimmte coloristische oder Echtheitseigenschaften zu verbessern, vorteilhaft sein, Gemische von Verbindungen der allgemeinen Formel I herzustellen. Dies kann im einfachsten Fall dadurch erreicht werden, daß man zwei oder mehrere Verbindungen der allgemeinen Formel I mechanisch mischt; dieses Vorgehen dient im allgemeinen dazu, eine bestimmte Farbnuance einzustellen.

Man kann aber auch sogenannte Mischkristalle herstellen. Ein Mischkristall oder eine feste Lösung liegt dann vor, wenn eine oder mehrere Verbindungen in das Kristallgitter einer "Wirtsverbindung" eingebaut werden. Das Röntgenbeugungsdiagramm dieses Mischkristalls zeigt dann nur die Reflexe der "Wirtsverbindung", während das Röntgenbeugungsdiagramm der entsprechenden mechanischen Mischung die Reflexe aller beteiligten Verbindungen aufweist. Ein solches Vorgehen wählt man, wenn neben den coloristischen auch rheologische bzw. Echtheitseigenschaften beeinflußt werden sollen.

Die Herstellung von Mischkristallen kann im Falle der Verbindungen der allgemeinen Formel I, worin R zusätzlich die Bedeutung Wasserstoff hat, auf verschiedenen Wegen erfolgen:

Zum einen kann man zwei oder mehrere Verbindungen der allgemeinen Formel I, beispielsweise in 96 bis 100 %iger Schwefelsäure lösen und die erhaltene Lösung durch Eingießen in Wasser hydrolysieren. Zum anderen kann man eine Mischung aus zwei oder mehreren 3-Amino-2-methyl-4-oxochinazolinen (vgl.

die vorstehend genannte allgemeine Formel III) mit einem 1,2-Diketon umsetzen.

Materialien, die mit den Verbindungen der allgemeinen Formel I gefärbt bzw. pigmentiert werden können, sind beispielsweise natürliche Harze oder Kunstharze, wie Polymerisations- oder Polykondensationsharze, aber auch Celluloseether oder -ester.

Die nachstehenden Beispsiele dienen zur Erläuterung der Erfindung, ohne sie darauf zu beschränken.

Die in den nachfolgenden Beispielen aufgeführten Teile sind Gewichtsteile; bei Angaben von Prozenten handelt es sich um Gewichtsprozente.

## Experimenteller Teil

Allgemeine Vorschrift zur Herstelung der 3-Amino-2-methyl-4-oxochinazoline der allgemeinen Formel III:

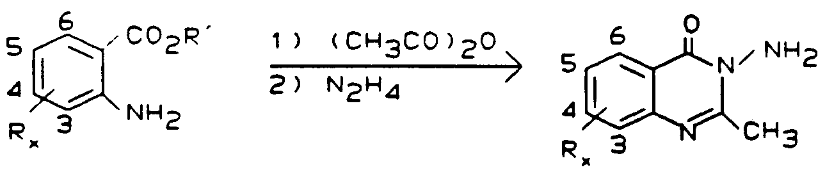


a Teile des Anthranilsäurederivats werden in b Teilen Essigsäureanhydrid 3 bis 5 Stunden zum Sieden erhitzt. Anschließend wird das Lösemittel entfernt und der Rückstand über Kaliumhydroxid im Exsikkator getrocknet. Die so erhaltene Substanz wird mit c ml Methanol verrührt und unter Kühlung werden bei 10 bis 15 ° C d Teile Hydrazinhydrat zugetropft. Man hält anschließend 30 Minuten bei 20 ° C, 30 Minuten bei 40 ° C und 30 Minuten bei Siedetemperatur. Anschließend wird bei 20 ° C abgesaugt, der Rückstand mit wenig Methanol gewaschen und bei 60 ° C im Umluftschrank getrocknet. Man erhält X Teile des 3-Amino-2-methyl-4-oxochinazolins (y % der Theorie, bezogen auf das Anthranilsäurederviat), das ohne weitere Reinigung zur Umsetzung mit den 1,2-Diketonen verwendet werden kann.

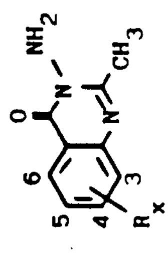

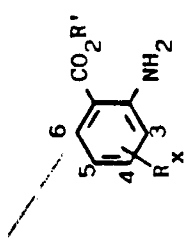

| Versuch | R | R' | a | b | c | d | X | y% | | R |
|---|---|---|---|---|---|---|---|---|---|---|
| 1 | 5-Cl | H | 85,8 | 300 | 300 | 125 | 66,7 | 64 | 1 | 5-Cl |
| 2 | 4-Cl | $CH_3$ | 92,8 | 300 | 150 | 125 | 95,7 | 91 | 2 | 4-Cl |
| 3 | 3,5-$Cl_2$ | H | 80,0 | 240 | 300 | 125 | 91,4 | 95 | 3 | 3,5-$Cl_2$ |
| 4 | 3,4,5-$Cl_3$ | H | 120,3 | 300 | 1200 | 125 | 123,9 | 89 | 4 | 3,4,5-$Cl_3$ |
| 5 | 4-$CF_3$ | H | 102,5 | 300 | 500 | 125 | 73,3 | 60 | 5 | 4-$CF_3$ |
| 6 | 5-Br | H | 22,3 | 60 | 60 | 25 | 10,2 | 40 | 6 | 5-Br |
| 7 | 3,5-$Br_2$ | $CH_3$ | 94,6 | 200 | 180 | 75 | 91,5 | 92 | 7 | 3,5-$Br_2$ |
| 8 | 6-$CH_3$ | H | 22,9 | 90 | 90 | 38 | 22,4 | 79 | 8 | 6-$CH_3$ |
| 9 | 5-$CH_3$ | H | 75,6 | 300 | 300 | 125 | 82,7 | 88 | 9 | 5-$CH_3$ |
| 10 | 3-$CH_3$ | H | 22,9 | 90 | 90 | 38 | 24,4 | 86 | 10 | 3-$CH_3$ |
| 11a | H | $C_2H_5$ | 82,6 | 300 | 300 | 125 | 70,5 | 80 | 11 | |
| 11b | H | $CH_3$ | 75,6 | 300 | 300 | 125 | 60,5 | 69 | 11 | |
| 12 | 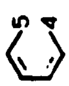 | H | 18,7 | 100 | 100 | 25 | 21,5 | 96 | 12 | 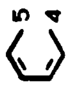 |

**Allgemeine Vorschrift zur Herstellung der 9-Oxo-1,9a,10-triaza-9-hydro-anthracene:**

Jeweils a Teile eines 3-Amino-2-methyl-4-oxochinazolins 1-12, das wie zuvor beschrieben hergestellt wurde, werden mit b Teilen 1,2-Diketon in c Teilen Lösemittel d Stunden bei einer Temperatur von T °C

am Wasserabscheider erhitzt. Man saugt bei 80 bis 100 °C ab, wäscht mit warmem Lösemittel nach, rührt den Filterkuchen in Dichlormethan an, saugt erneut ab, wäscht mit Dichlormethan und trocknet (60 °C, Umluftschrank) und erhält x Teile (y % der Theorie) des 9-Oxo-1,9a,10-triaza-9-hydro-anthracen-derivats. m Teile des so erhaltenen Produkts werden bei 15 bis 20 °C in n Teilen 96 bis 100 %iger Schwefelsäure gelöst bzw. suspendiert und 1 Stunde bei dieser Temperatur belassen. Man gießt den Ansatz auf o Teile Eiswasser, erhitzt anschließend für 2 Stunden auf 90 °C, saugt ab, wäscht neutral, trocknet und erhält p Teile (q %) 9-Oxo-1,9a,10-triaza-9-hydro-anthracen-derivat zurück.

Abkürzungen für die in nachfolgender Tabelle verwendeten 1,2-Diketone und Lösemittel

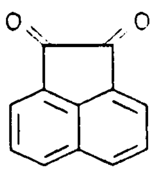

ANC

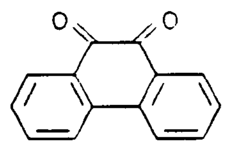

PC

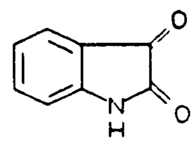

IS

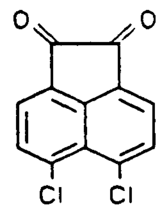

$Cl_2$-ANC

| | |
|---|---|
| ANC = | Acenaphthenchinon |
| PC = | Phenanthrenchinon |
| IS = | Isatin |
| o-DCB = | 1,2-Dichlorbenzol |
| DT = | Dowtherm A = 76,5 % Diphenylether, 23,5 % Diphenyl |
| DIN = | Diisopropylnaphthalin (Isomerengemisch) |
| CN = | 1-Chlornaphthalin |

| Vers. | a | R | 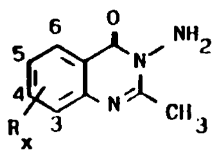 | b | 1,2-Diketon | c | Lösemittel | d | T °C | X | y% | Produkt | m | n | o | p | q% |
|---|---|---|---|---|---|---|---|---|---|---|---|---|---|---|---|---|---|
| 13 | 20,9 | 5-Cl | 1 | 20,0 | ANC | 500 | o-DCB | 6 | reflux | 32,7 | 92 | 13 | 30,0 | 300 | 2400 | 29,9 | 99 |
| 14 | 20.9 | 5-Cl | 1 | 22,9 | PC | 500 | o-DCB | 6 | reflux | 29,8 | 78 | 14 | 25,0 | 250 | 2800 | 24,3 | 97 |
| 15 | 20,9 | 4-Cl | 2 | 20,9 | ANC | 500 | o-DCB | 6 | reflux | 27,7 | 78 | 15 | 25,0 | 250 | 2000 | 23,7 | 95 |
| 16 | 20,9 | 4-Cl | 2 | 22,9 | PC | 500 | o-DCB | 6 | reflux | 24,8 | 65 | 16 | 24.0 | 250 | 2000 | 23,7 | 96 |
| 17 | 20,9 | 4-Cl | 2 | 16,2 | IS | 500 | o-DCB | 6 | reflux | 6,0 | 19 | 17 | 5,5 | 100 | 800 | 5,0 | 91 |
| 18 | 24,4 | 3,5-$Cl_2$ | 3 | 20.0 | ANC | 500 | o-DCB | 6 | reflux | 31,2 | 80 | 18 | 28,0 | 280 | 2800 | 24,1 | 86 |
| 19 | 24,4 | 3,5-$Cl_2$ | 3 | 22,9 | PC | 500 | o-DCB | 6 | reflux | 28,2 | 68 | 19 | 25,0 | 250 | 2500 | 16,7 | 67 |
| 20 | 24,4 | 3,5-$Cl_2$ | 3 | 16,2 | IS | 500 | o-DCB | 6 | reflux | 4,2 | 12 | 20 | 3,5 | 150 | 1500 | 2,4 | 69 |
| 21 | 27.8 | 3,4,5-$Cl_3$ | 4 | 20,0 | ANC | 500 | o-DCB | 7 | reflux | 30,2 | 71 | 21 | 27,7 | 380 | 3000 | 27,4 | 99 |
| 22 | 27,8 | 3,4,5-$Cl_3$ | 4 | 22,9 | PC | 500 | o-DCB | 7 | reflux | 35,6 | 79 | 22 | 34,0 | 640 | 5000 | 32.0 | 94 |
| 23 | 24,3 | 4-$CF_3$ | 5 | 20,0 | ANC | 500 | o-DCB | 10 | reflux | 29,8 | 77 | 23 | 28,4 | 300 | 2400 | 27,3 | 96 |
| 24 | 24,3 | 4-$CF_3$ | 5 | 22,9 | PC | 500 | o-DCB | 10 | reflux | 14,8 | 36 | 24 | 13,7 | 150 | 1200 | 12,4 | 91 |
| 25 | 8,46 | 5-Br | 6 | 6,70 | ANC | 170 | o-DCB | 10 | reflux | 10,6 | 80 | 25 | 10,6 | 150 | 1200 | 10,2 | 96 |
| 26 | 16,6 | 3,5-$Br_2$ | 7 | 10,0 | ANC | 250 | o-DCB | 7 | reflux | 17,9 | 75 | 26 | 17,6 | 200 | 1600 | 17,4 | 99 |
| 27 | 16,6 | 3,5-$Br_2$ | 7 | 11,4 | PC | 250 | o-DCB | 7 | reflux | 17,6 | 70 | 27 | 17,6 | 300 | 2600 | 17,2 | 98 |

| Vers. 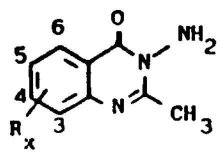 a | R | | b | 1,2-Diketon | c | Lösemittel | d | T °C | X | y% | Produkt | m | n | o | p | q% |
|---|---|---|---|---|---|---|---|---|---|---|---|---|---|---|---|---|
| 28 9,45 | 6-$CH_3$ | 8 | 10,0 | ANC | 250 | o-DCB | 10 | reflux | 5,4 | 32 | 28 | 5,4 | 100 | 800 | 5.1 | 94 |
| 29 9,45 | 6-$CH_3$ | 8 | 11,4 | PC | 250 | o-DCB | 10 | reflux | 8,7 | 48 | 29 | 8,4 | 150 | 1200 | 8,0 | 95 |
| 30 18,9 | 5-$CH_3$ | 9 | 20.0 | ANC | 500 | o-DCB | 6 | reflux | 27,1 | 81 | 30 | 25,0 | 250 | 2500 | 24,4 | 98 |
| 31 18,9 | 5-$CH_3$ | 9 | 22,9 | PC | 500 | o-DCB | 6 | reflux | 20,7 | 57 | 31 | 18,0 | 280 | 2800 | 17,7 | 98 |
| 32 9,5 | 3-$CH_3$ | 10 | 10,0 | ANC | 250 | o-DCB | 6 | reflux | 14,2 | 85 | 32 | 13,5 | 200 | 1600 | 13,2 | 98 |
| 33 14,3 | 3-$CH_3$ | 10 | 11,5 | PC | 250 | o-DCB | 6 | reflux | 7,7 | 43 | 33 | 7,2 | 150 | 1200 | 6,5 | 90 |
| 36 6,92 | H | 11 | 10,5 | $Cl_2$-ANC | 200 | o-DCB | 6 | reflux | 7,6 | 49 | 36 | 6,9 | 150 | 1200 | 6,1 | 88 |
| 37 22,5 | 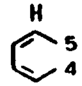 | 12 | 20,0 | ANC | 500 | o-DCB | 6 | reflux | 27,2 | 73 | 37 | 25,0 | 250 | 2000 | 23,7 | 95 |

<u>**13**</u>

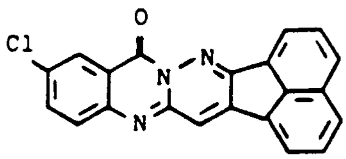

$C_{21}H_{10}N_3OCl$
gef. C 70,0% H 2,7% N 11,6%
gelb

<u>**14**</u>

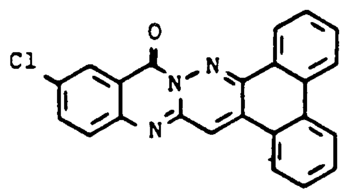

$C_{23}H_{12}N_3OCl$
gef. C 70,1% H 3,1% N 10,8%
orange

<u>**15**</u>

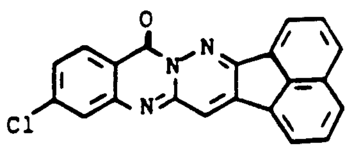

$C_{21}H_{10}N_3OCl$
gef. C 71,4% H 2,8% N 11,9%
grünstichig gelb

<u>**16**</u>

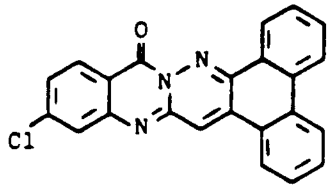

$C_{23}H_{12}N_3OCl$
gef. C 72,3% H 3,0% N 11,1% Cl 9,2%
orange

<u>**17**</u>

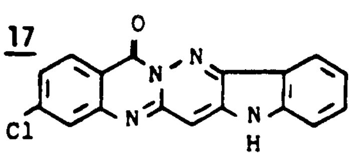

+

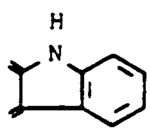

$C_{17}H_9N_4OCl$ -
gef. C 60,8% H 2,8% N 16,8% Cl 10,5%
orange

<u>**18**</u>

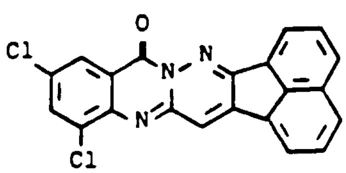

$C_{21}H_9N_3OCl_2$
gef. C 64,8% H 2,3% N 10,8%
gelb

**19**

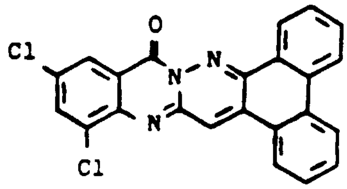

$C_{23}H_{11}N_3OCl_2$
gef. C 66,1% H 2,7% N 10,0%
orange

**20**

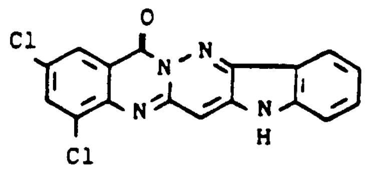

+

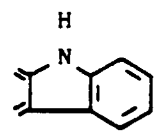

$C_{17}H_8N_4OCl_2$
gef. C 55,2% H 2,1% N 13,8%
braunes Orange

**21**

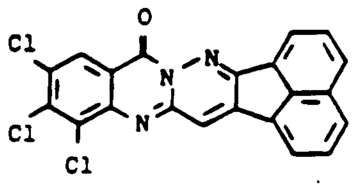

$C_{21}H_8N_3OCl_3$
gef. C 59,5% H 1,7% N 9,4% Cl 25,0%
gelb

**22**

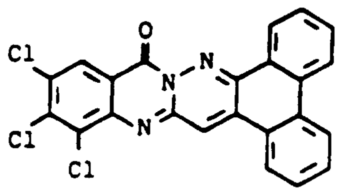

$C_{23}H_{10}N_3OCl_3$
gef. C 61,4% H 2,3% N 8,7% Cl 23,6%
orange

**23**

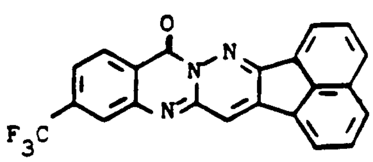

$C_{22}H_{10}N_3OF_3$
gef. C 67,9% H 2,6% N 10,8%
grünstichiges Gelb

**24**

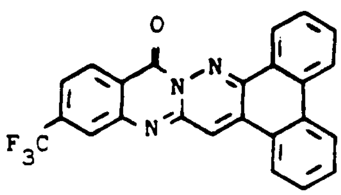

$C_{24}H_{12}N_3OF_3$
gef. C 69,0% H 2,9% N 9,6%
orange

**25**

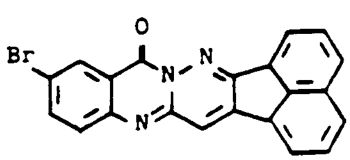

$C_{21}H_{10}N_3OBr$
gef. C 64,0% H 2,8% N 9,9%
gelb

26 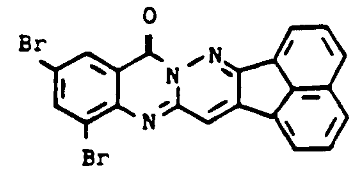

$C_{21}H_9N_3OBr_2$
gef. C 53,0% H 1,9% N 8,8%
gelb

27 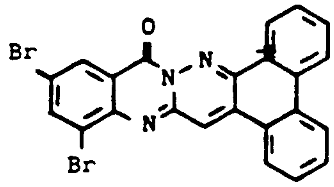

$C_{23}H_{11}N_3OBr_2$
gef. C 54,4% H 2,1% N 8,8% Br 31,9%
orange

28 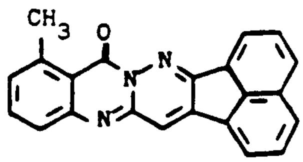

$C_{22}H_{13}N_3O$
gef. C 78,1% H 3,8% N 12,5%
gelb

29 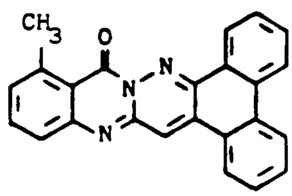

$C_{24}H_{15}N_3O$
gef. C 77,6% H 3,8% N 12,0%
orange

30 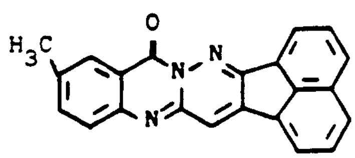

$C_{22}H_{13}N_3O$
gef. C 74,6% H 3,7% N 11,7%
gelb

31 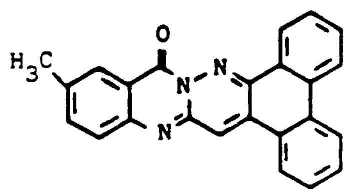

$C_{24}H_{15}N_3O$
gef. C 79,1% H 4,1% N 10,7%
orange

32 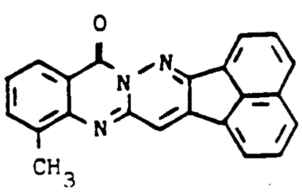

$C_{22}H_{13}N_3O$
gef C 78,2% H 3,8% N 12,5%
gelb

33

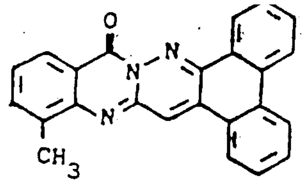

$C_{24}H_{15}N$
gef. C 78,1% H 4,2% N 11,3%
orange

36

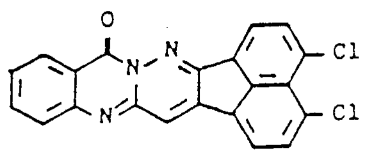

$C_{21}H_{9}N_{3}OCl_{2}$
gef. C 64,2% H 2,4% N 10,5% Cl 18,3%
gelb

37

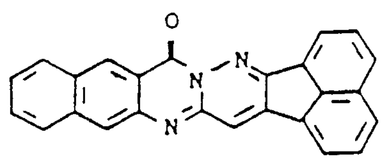

$C_{25}H_{13}N_{3}O$
gef. C 79,2% H 3,5% N 11,2%
orange

Die Struktur der zuvor aufgeführten Verbindungen 13 bis 37 wird weiterhin durch $^{1}$H-NMR-Spektroskopie bestätigt.

**Versuch 38:**

14,2 Teile I (R = 5-Cl) 13 und 3,2 Teile I (R = H) 34

34

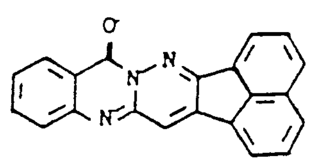

gelb

werden in 250 Teile 96 %iger Schwefelsäure bei 20 °C gelöst. Die erhaltene Lösung läßt man in 2500 Teile Wasser von 0 °C einlaufen. Anschließend erhitzt man die entstandene Suspension 2 Stunden auf 90 °C. Dann saugt man ab, wäscht neutral und trocknet. Man erhält 8,5 Teile (98 % der Theorie) eines gelben Pigments, dessen Röntgenbeugungsdiagramm nur die Reflexe der Verbindung 13 zeigt.

**Versuch 39:**

9,76 Teile 3 und 2,09 Teile 1 sowie 10,1 Teile Acenaphthenchinon werden in 300 ml o-Dichlorbenzol 7 Stunden am Wasserabscheider zum Sieden erhitzt. Anschließend saugt man bei 80 °C ab, wäscht mit warmem o-Dichlorbenzol, rührt den Rückstand in Dichlormethan an, saugt ab, trocknet und erhält 16,5 Teile (86 % der Theorie) eines Mischkristalls, der im Röntgenbeugungsdiagramm nur die Reflexe der Verbindung 18 zeigt.

**Versuch 40:**

Die erhaltenen Pigmente werden nach Einarbeiten in ein TSA-Lacksystem miteinander verglichen.

a) 30,0 Teile der Verbindung 18 werden in 400 Teilen 100 %iger Schwefelsäure bei 10 bis 15 °C gelöst und diese Lösung durch Eingießen in 3200 Teile Wasser von 0 °C hydrolysiert. Man erhitzt die

entstandene Suspension noch 2 Stunden auf 90 °C, saugt ab, wäscht neutral und trocknet. Man erhält 29,3 Teile (98 % d. Th.) 18 als gelbes Pigment zurück.

b) 15,0 Teile der Verbindung 18, wie sie nach Versuch 18 erhalten wurde, werden in einer Rührwerkskugelmühle mit 140 Teilen Wasser und 10 Teilen Isobutanol in Gegenwart von Glasperlen (2 mm Durchmesser) 3 Stunden bei Raumtemperatur gemahlen. Der Alkohol wird mit Wasserdampf abdestilliert und die verbleibende Suspension abgesaugt, gewaschen und getrocknet. Man erhält 15,0 Teile eines gelben Pigments zurück, das in seinen coloristischen Eigenschaften mit dem nach a) erhaltenen Pigment vergleichbar ist.

c) 15,0 Teile der Verbindung 18, wie sie nach Versuch 18 erhalten wurde, werden in einer Rollmühle mit 30 Teilen Natriumsulfat in Gegenwart von Porzellankugeln 4 Stunden gemahlen. Das Mahlgut wird mit 600 Teilen Wasser behandelt und der Rückstand abgesaugt, salzfrei gewaschen und getrocknet. Man erhält 29,2 Teile (98 % d. Th.) eines gelben Pigments, welches deutlich transparenter als das nach a) erhaltene ist.

## Patentansprüche

**1.** Derivate des 9-Oxo-1,9a,10-triaza-9-hydro-anthracens der allgemeinen Formel I

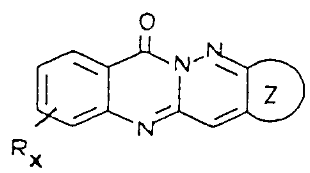

(I) ,

in welcher (Formel I) R ein Halogenatom, eine der Gruppen Alkyl-$C_1$-$C_4$, Alkoxy-$C_1$-$C_4$, Trihalogenmethyl, -CO-$NH_2$, -CO-NH-(alkyl-$C_1$-$C_4$) oder -CO-N-(alkyl-$C_1$-$C_4$)$_2$ oder eine der über das Brückenglied -S-, -SO- oder -$SO_2$- gebundenen Gruppen Phenyl, Alkyl-$C_1$-$C_4$, -$NH_2$, -NH-(alkyl-$C_1$-$C_4$), -N-(alkyl-$C_1$-$C_4$)$_2$ oder -alkylen-$C_1$-$C_4$-$OSO_3H$ oder einen ankondensierten 4- bis 7-gliedrigen Iso- oder Heterocyclus darstellt, welcher selbst Bestandteil eines polycyclischen Systems aus der Reihe Naphthalin, Acenaphthen, Phenanthren und Indol sein kann, x eine ganze Zahl von 1 bis 4 bedeutet, wobei für $x > 1$ R gleich oder verschieden sein kann, und Z einen ankondensierten 4- bis 7-gliedrigen Iso- oder Heterocyclus aus der Reihe Cyclobutan, Cyclopentan, Thiophen, Pyrrol, Furan, Cyclohexan, Pyridin, Pyrimidin und Cycloheptan darstellt, welcher selbst Bestandteil eines polycyclischen Systems aus der Reihe Naphthalin, Acenaphthen, Phenanthren und Indol sein kann, wobei jeweils der genannte 4- bis 7-gliedrige Iso- oder Heterocyclus und jeweils das genannte polycyclische System durch Halogenatome, eine der Gruppen Alkyl-$C_1$-$C_4$, Alkoxy-$C_1$-$C_4$, Trihalogenmethyl, -CO-$NH_2$, -CO-NH-(alkyl-$C_1$-$C_4$) oder -CO-N-(alkyl-$C_1$-$C_4$)$_2$ oder über das Brückenglied -S-, -SO- oder -$SO_2$-gebundene Phenyl-, Alkyl-$C_1$-$C_4$, -$NH_2$-, -NH-(alkyl-$C_1$-$C_4$)-, -N-(alkyl-$C_1$-$C_4$)$_2$- oder -alkylen-$C_1$-$C_4$-$OSO_3H$-Gruppen substituiert sein kann.

**2.** Derivate des 9-Oxo-1,9a,10-triaza-9-hydro-anthracens der allgemeinen Formel I

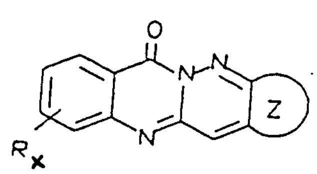

(I) ,

in welcher (Formel I) R ein Chlor- oder Bromatom, eine der Gruppen Alkyl-$C_1$-$C_4$, Alkoxy-$C_1$-$C_4$, Trifluoromethyl, Trichloromethyl, -CO-$NH_2$, -CO-NH-(alkyl-$C_1$-$C_4$) oder -CO-N-(alkyl-$C_1$-$C_4$)$_2$ oder eine der über das Brückenglied -S-,-SO- oder -$SO_2$- gebundenen Gruppen Phenyl, Alkyl-$C_1$-$C_4$, -$NH_2$, -NH-(alkyl-$C_1$-$C_4$), -N-(alkyl-$C_1$-$C_4$)$_2$ oder -alkylen-$C_1$-$C_4$-$OSO_3H$ oder einen ankondensierten 4- bis 7-gliedrigen Iso- oder Heterocyclus aus der Reihe Cyclobutan, Cyclopentan-, Thiophen, Pyrrol, Furan, Cyclohexan, Pyridin, Pyrimidin und Cycloheptan bedeutet, welcher selbst Bestandteil eines polycyclischen Systems aus der Reihe Naphthalin, Acenaphthen, Phenanthren und Indol sein kann, x eine ganze Zahl von 1 bis 4 bedeutet, wobei für x > 1 R gleich oder verschieden sein kann, und Z einen ankondensierten 4- bis 7-gliedrigen Iso- oder Heterocyclus aus der Reihe Cyclobutan, Cyclopentan, Thiophen, Pyrrol, Furan, Cyclohexan, Pyridin, Pyrimidin und Cycloheptan bedeutet, welcher selbst Bestandteil eines polycyclischen Systems aus der Reihe Naphthalin, Acenaphthen, Phenanthren und Indol sein kann, wobei jeweils der genannte 4- bis 7-gliedrige Iso- oder Heterocyclus und jeweils das genannte polycyclische System durch Chlor- oder Bromatome, eine der Gruppen Alkyl-$C_1$-$C_4$, Alkoxy-$C_1$-$C_4$, Trifluormethyl, Trichlormethyl, -CO-$NH_2$, -CO-NH-(alkyl-$C_1$-$C_4$) oder -CO-N-(alkyl-$C_1$-$C_4$)$_2$ oder eine der über das Brückenglied -S-, -SO-oder -$SO_2$- gebundenen Gruppen Phenyl, Alkyl-$C_1$-$C_4$, -$NH_2$, -NH-(alkyl-$C_1$-$C_4$), -N-(alkyl-$C_1$-$C_4$)$_2$ oder -alkylen-$C_1$-$C_4$-$OSO_3H$ substituiert sein können.

**3.** Mischkristalle, bestehend aus mindestens zwei Verbindungen der in Anspruch 1 angegebenen allgemeinen Formel I, worin R zusätzlich die Bedeutung Wasserstoff hat, dadurch gekennzeichnet, daß mindestens eine dieser Verbindungen im Kristallgitter einer der anderen Verbindungen vorliegt.

**4.** Verfahren zur Herstellung von Derivaten des 9-Oxo-1,9a,10-triaza-9-hydro-anthracens, der in Anspruch 1 angegebenen allgemeinen Formel I, dadurch gekennzeichnet, daß man Anthranilsäurederivate der allgemeinen Formel II

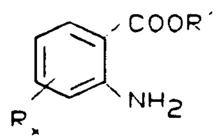

(II)

in welcher R′ ein Wasserstoffatom oder eine Alkyl$_{C_1-C_4}$ oder Arylgruppe darstellt, R und x die in Anspruch 1 genannten Bedeutungen haben, zunächst mit 1 bis etwa 50 Mol Acetanhydrid bei Temperaturen von etwa 50 bis etwa 180 °C, bei Normaldruck oder bei erhöhtem oder vermindertem Druck, und dann mit 1 bis etwa 10 Mol Hydrazin bei Temperaturen von etwa 0 bis etwa 120 °C und anschließend das erhaltene Reaktionsprodukt der allgemeinen Formel III

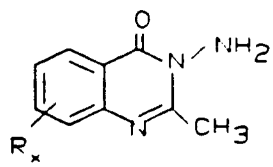

(III)

in welcher R und x die in Anspruch 1 genannten Bedeutungen haben, mit 1 bis etwa 2 Mol 1,2-Diketon der allgemeinen Formel IV

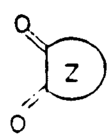

(IV)

in welcher Z die in Anspruch 1 genannten Bedeutungen hat, bei Temperturen von etwa 140 bis 240 °C in einem den Reaktionsteilnehmern gegenüber inerten organischen Lösemittel unter gleichzeitiger Entfernung des bei der Reaktion entstehenden Wassers umsetzt.

**5.** Verfahren zur Herstellung von Derivaten des 9-Oxo-1,9a,10-triaza-9-hydro-anthracens, der in Anspruch 1 angegebenen allgemeinen Formel I, dadurch gekennzeichnet, daß man Anthranilsäurederivate der allgemeinen Formel II

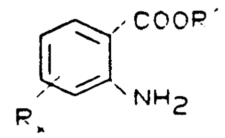

(II)

in welcher R' ein Wasserstoffatom oder eine Alkyl$C_1$-$C_4$, Phenyl- oder Toluylgruppe darstellt, R und x die in Anspruch 2 angegebenen Bedeutungen haben, zunächst mit etwa 4 bis etwa 15 Mol Acetanhydrid bei der Siedetemperatur des Acetanhydrids bei Normalbedingungen, dann mit etwa 2 bis etwa 6 Mol Hydrazin bei Temperaturen von etwa 15 bis etwa 70 °C und anschließend das erhaltene Reaktionsprodukt der allgemeinen Formel III

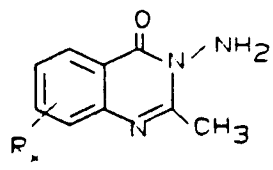

(III)

in welcher R und x die in Anspruch 2 angegebenen Bedeutungen haben, mit etwa 1,1 bis etwa 1,2 Mol 1,2-Diketon der allgemeinen Formel IV

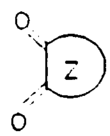

(IV)

in welcher Z die in Anspruch 2 angegebenen Bedeutungen hat, bei Temperaturen von etwa 160 bis etwa 180 °C in einem den Reaktionsteilnehmern gegenüber inerten organischen Lösemittel unter gleichzeitiger Entfernung des bei der Reaktion entstehenden Wassers umsetzt.

**6.** Verfahren nach mindestens einem der Ansprüche 4 und 5, dadurch gekennzeichnet, daß man die Umsetzung mit dem Diketon in o-Dichlorbenzol, Diisopropylnaphthalin, Chlornaphthalin oder in einer Mischung aus 76,5 % Diphenylether und 23,5 % Diphenyl als inertem Lösemittel durchführt.

**7.** Verfahren zur Herstellung der in Anspruch 3 beanspruchten Mischkristalle, dadurch gekennzeichnet, daß man mindestens zwei verschiedene 3-Amino-2-methyl-4-oxochinazoline der in Anspruch 4 genannten allgemeinen Formel III mit 1 bis etwa 2 Mol 1,2-Diketon der in Anspruch 4 genannten allgemeinen Formel IV pro Mol 3-Amino-2-methyl-4-oxochinazolin bei Temperaturen von etwa 140 bis etwa 240 °C umsetzt.

**8.** Verfahren zur Herstellung der in Anspruch 3 beanspruchten Mischkristalle, dadurch gekennzeichnet, daß man mindestens zwei verschiedene Verbindungen der in Anspruch 1 angegebenen allgemeinen Formel I in einer gegenüber den Verbindungen inerten Säure löst und die erhaltene Lösung durch Eingießen in Wasser hydrolysiert.

**9.** Verfahren nach Anspruch 8, dadurch gekennzeichnet, daß man die Verbindungen der allgemeinen Formel I in der etwa 5- bis etwa 10-fachen Gewichtsmenge 96 bis 100 %iger Schwefelsäure löst.

**10.** Verwendung der in mindestens einem der Ansprüche 1, 2 und 3 beschriebenen Verbindungen zum Einfärben von natürlichen oder synthetischen Materialien.

## Claims

1. A derivative of 9-oxo-1,9a,10-triaza-9-hydroanthracene of the general formula I

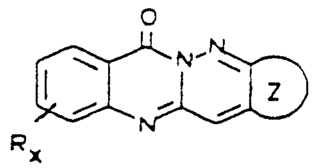

(I),

in which (formula I) R denotes a halogen atom, one of the groups alkyl-$C_1$-$C_4$, alkoxy-$C_1$-$C_4$, trihalogenomethyl, -CO-$NH_2$, -CO-NH(alkyl-$C_1$-$C_4$) or -CO-N(alkyl-$C_1$-$C_4$)$_2$ or one of the groups phenyl, alkyl-$C_1$-$C_4$, -$NH_2$, -NH(alkyl-$C_1$-$C_4$), -N(alkyl-$C_1$-$C_4$)$_2$ or -alkylene-$C_1$-$C_4$-$OSO_3H$, bound via the bridge member -S-, -SO- or -$SO_2$-, or a fused-on 4- to 7-membered isocycle or heterocycle, which itself can be part of a polycyclic system, from the series consisting of naphthalene, acenaphthene, phenanthrene and indole, x denotes an integer from 1 to 4, where for x > 1 R can be identical or different, and Z denotes a fused-on 4- to 7-membered isocycle or heterocycle from the series consisting of cyclobutane, cyclopentane, thiophene, pyrrole, furan, cyclohexane, pyridine, pyrimidine and cycloheptane, which itself can be part of a polycyclic system, from the series consisting of naphthalene, acenaphthene, phenanthrene and indole, in which each of the 4- to 7-membered isocycles or heterocycles mentioned and each of the polycyclic systems mentioned can be substituted by halogen atoms, one of the groups alkyl-$C_1$-$C_4$, alkoxy-$C_1$-$C_4$, trihalogenomethyl, -CO-$NH_2$, -CO-NH(alkyl-$C_1$-$C_4$) or -CO-N(alkyl-$C_1$-$C_4$)$_2$ or phenyl, alkyl-$C_1$-$C_4$, -$NH_2$, -NH(alkyl-$C_1$-$C_4$), -N(alkyl-$C_1$-$C_4$)$_2$ or -alkylene-$C_1$-$C_4$-$OSO_3H$ groups which are bound via the bridge member -S-, -SO- or -$SO_2$-.

2. A derivative of 9-oxo-1,9a,10-triaza-9-hydroanthracene of the general formula I

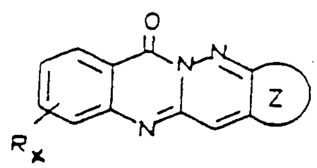

(I),

in which (formula I) R denotes a chlorine or bromine atom, one of the groups alkyl-$C_1$-$C_4$, alkoxy-$C_1$-$C_4$, trifluoromethyl, trichloromethyl, -CO-$NH_2$, -CO-NH(alkyl-$C_1$-$C_4$) or -CO-N(alkyl-$C_1$-$C_4$)$_2$ or one of the groups phenyl, alkyl-$C_1$-$C_4$, -$NH_2$, -NH(alkyl-$C_1$-$C_4$), -N(alkyl-$C_1$-$C_4$)$_2$ or -alkylene-$C_1$-$C_4$-$OSO_3H$, bound via the bridge member -S-, -SO- or -$SO_2$-, or a fused-on 4- to 7-membered isocycle or heterocycle from the series consisting of cyclobutane, cyclopentane, thiophene, pyrrole, furan, cyclohexane, pyridine, pyrimidine and cycloheptane, which itself can be part of a polycyclic system, from the series consisting of naphthalene, acenaphthene, phenanthrene and indole, x denotes an integer from 1 to 4, where for x > 1 R can be identical or different, and Z denotes a fused-on 4- to 7-membered isocycle or heterocycle from the series consisting of cyclobutane, cyclopentane, thiophene, pyrrole, furan, cyclohexane, pyridine, pyrimidine and cycloheptane, which itself can be part of a polycyclic system, from the series consisting of naphthalene, acenaphthene, phenanthrene and indole, in which each of the 4- to 7-membered isocycles or heterocycles mentioned and each of the polycyclic systems mentioned can be substituted by chlorine or bromine atoms, one of the groups alkyl-$C_1$-$C_4$, alkoxy-$C_1$-$C_4$, trifluoromethyl, trichloromethyl, -CO-$NH_2$, -CO-NH(alkyl-$C_1$-$C_4$) or -CO-N(alkyl-$C_1$-$C_4$)$_2$ or one of the groups phenyl, alkyl-$C_1$-$C_4$, -$NH_2$, -NH(alkyl-$C_1$-$C_4$), -N(alkyl-$C_1$-$C_4$)$_2$ or -alkylene-$C_1$-$C_4$-$OSO_3H$ which are bound via the bridge member -S-, -SO- or -$SO_2$-.

3. A mixed crystal consisting of at least two compounds of the general formula I mentioned in claim 1 in which R additionally denotes hydrogen, wherein at least one of these compounds is present in the crystal lattice of one of the other compounds.

4. A process for the preparation of a derivative of 9-oxo-1,9a,10-triaza-9-hydroanthracene of the general formula I mentioned in claim 1,
which comprises reacting anthranilic acid derivatives of the general formula II

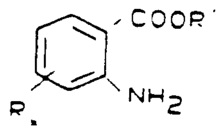

(II)

in which R' represents a hydrogen atom or an alkyl-$C_1$-$C_4$ or aryl group, R and x have the meanings mentioned in claim 1, first with 1 to about 50 mol of acetic anhydride at temperatures of about 50 to about 180°C, at atmospheric pressure or at elevated or reduced pressure, and then reacting the product with 1 to about 10 mol of hydrazine at temperatures of about 0 to about 120°C, subsequently reacting the reaction product obtained of the general formula III

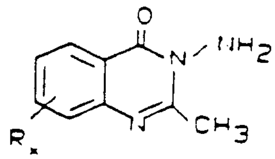

(III)

in which R and x have the meanings mentioned in claim 1, with 1 to about 2 mol of 1,2-diketone of the general formula IV

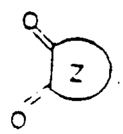

(IV)

in which Z has the meanings mentioned in claim 1, at temperatures of about 140 to 240°C in an organic solvent which is inert towards the reactants while simultaneously removing the water formed in the reaction.

5. The process for the preparation of a derivative of 9-oxo-1,9a,10-triaza-9-hydroanthracene of the general formula I mentioned in claim 1,
which comprises reacting anthranilic acid derivatives of the general formula II

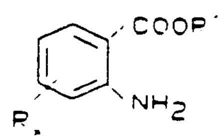

(II)

in which R' represents a hydrogen atom or an alkyl-$C_1$-$C_4$,phenyl or toluyl group, R and x have the meanings mentioned in claim 2, first with about 4 to about 15 mol of acetic anhydride at the boiling temperature of acetic anhydride under standard conditions, and then reacting the product with about 2 to about 6 mol of hydrazine at temperatures of about 15 to about 70°C, subsequently reacting the reaction product obtained of the general formula III

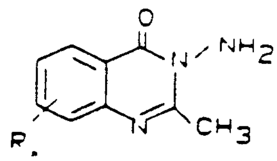

(III)

in which R and x have the meanings mentioned in claim 2, with about 1.1 to about 1.2 mol of 1,2-diketone of the general formula IV

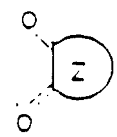

(IV)

in which Z has the meanings mentioned in claim 2, at temperatures of about 160 to about 180 °C in an organic solvent which is inert towards the reactants while simultaneously removing the water formed in the reaction.

**6.** The process as claimed in at least one of claims 4 and 5, wherein the reaction with the diketone is carried out in o-dichlorobenzene, diisopropylnaphthalene, chloronaphthalene or in a mixture consisting of 76.5% of diphenyl ether and 23.5% of biphenyl as the inert solvent.

**7.** A process for the preparation of a mixed crystal as claimed in claim 3, which comprises reacting at least two different 3-amino-2-methyl-4-oxoquinazolines of the general formula III mentioned in claim 4 with 1 to about 2 mol of 1,2-diketone of the general formula IV mentioned in claim 4 per mole of 3-amino-2-methyl-4-oxoquinazoline at temperatures of about 140 to about 240 °C.

**8.** A process for the preparation of a mixed crystal as claimed in claim 3, which comprises dissolving at least two different compounds of the general formula I mentioned in claim 1 in an acid which is inert towards the compounds and hydrolyzing the resulting solution by pouring it into water.

**9.** The process as claimed in claim 8, wherein the compounds of the general formula I are dissolved in about 5 to about 10 times the amount by weight of 96 to 100% strength sulfuric acid.

**10.** Use of the compounds described in at least one of claims 1, 2 and 3 for the coloring of natural or synthetic materials.

**Revendications**

**1.** Dérivés du 9-oxo-1,9a,10-triaza-9-hydroanthracène de formule générale I

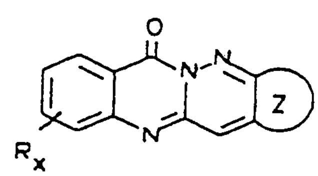

(I) ,

dans laquelle (formule I) R signifie un atome d'halogène, un des groupements alkyle en $C_1$-$C_4$, alkoxy en $C_1$-$C_4$, méthyle trihalogéné, -CO-$NH_2$, -CO-NH-(alkyle en $C_1$-$C_4$) ou -CO-N-(alkyle en $C_1$-$C_4$)$_2$ ou un des groupements phényle, alkyle en $C_1$-$C_4$, -$NH_2$, -NH-(alkyle en $C_1$-$C_4$), -N-(alkyle en $C_1$-$C_4$)$_2$ ou -(alcylène en $C_1$-$C_4$)-$OSO_3H$, lié par un chaînon -S-, -SO- ou -$SO_2$-, ou un isocycle ou hétérocycle, condensé, de 4 à 7 chaînons, lui-même pouvant être un constituant d'un système polycyclique de la

série naphtalène, acénaphtène, phénanthrène et indole, x signifie un nombre entier compris entre 1 et 4, R pouvant être identique ou différent lorsque x > 1, et Z représente un isocycle ou hétérocycle condensé, de 4 à 7 chaînons de la série cyclobutane, cyclopentane, thiophène, pyrrole, furanne, cyclohexane, pyridine, pyrimidine et cycloheptane, lui-même pouvant être un constituant d'un système polycyclique de la série naphtalène, acénaphtène, phénanthrène et indole, suivant le cas, l'isocycle ou l'hétérocycle indiqué, de 4 à 7 chaînons, et suivant le cas, le système polycyclique indiqué pouvant être substitué par des atomes d'halogène, un des groupes alkyle en $C_1$-$C_4$, alkoxy en $C_1$-$C_4$, méthyle triohalogéné, -CO-$NH_2$, -CO-NH-(alkyle en $C_1$-$C_4$) et -CO-N-(alkyle en $C_1$-$C_4$)$_2$ ou des groupements phényle, alkyle en $C_1$-$C_4$, -$NH_2$, -NH-(alkyle en $C_1$-$C_4$), -N-(alkyle en $C_1$-$C_4$)$_2$ ou -(alcylène en $C_1$-$C_4$)-$OSO_3H$ liés par un chaînon de pont -S-, -SO- ou -$SO_2$-.

**2.** Dérivé du 9-oxo-1,9a,10-triaza-9-hydroanthracène de formule générale I

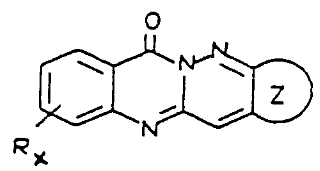

(I) ,

dans laquelle (formule I) R signifie un atome de chlore ou de brome, un des groupes alkyle en $C_1$-$C_4$, alkoxy en $C_1$-$C_4$, trifluorométhyle, trichlorométhyle, -CO-$NH_2$, -CO-NH-(alkyle en $C_1$-$C_4$) ou -CO-N-(alkyle en $C_1$-$C_4$)$_2$ ou un des groupes phényle, alkyle en $C_1$-$C_4$, -$NH_2$, -NH-(alkyle en $C_1$-$C_4$), -N-(alkyle en $C_1$-$C_4$)$_2$ ou -(alcylène en $C_1$-$C_4$)-$OSO_3H$ lié par le chaînon de pont -S-, -SO- ou -$SO_2$- ou bien un isocycle ou hétérocycle de 4 à 7 chaînons condensé de la série cyclobutane, cyclopentane, thiophène, pyrrole, furanne cyclohexane, pyridine, pyrimidine et cycloheptane, lui-même pouvant être un constituant d'un système polycyclique de la série naphtalène, acénaphtène, phénanthrène et indole, x signifie un nombre entier compris entre 1 et 4, R pouvant être identique ou différent lorsque x > 1, et Z signifie un isocycle ou un hétérocycle de 4 à 7 chaînons condensé de la série cyclobutane, cyclopentane, thiophène, pyrrole, furanne, cyclohexane, pyridine, pyrimidine et cycloheptane, lui-même pouvant être un constituant d'un système polycyclique de la série naphtalène, acénaphtène, phénanthrène et indole, suivant le cas les isocycles ou hétérocycles de 4 à 7 chaînons mentionnés et suivant le cas les systèmes polycycliques mentionnés pouvant être substitués par des atomes de chlore ou de brome, un des groupes alkyle en $C_1$-$C_4$, alkoxy en $C_1$-$C_4$, trifluorométhyle, trichlorométhyle, -CO-$NH_2$, -CO-NH-(alkyle en $C_1$-$C_4$) ou -CO-N-(alkyle en $C_1$-$C_4$)$_2$ ou un des groupes phényle, alkyle en $C_1$-$C_4$, -$NH_2$, -NH-(alkyle en $C_1$-$C_4$), -N-(alkyle en $C_1$-$C_4$)$_2$ ou -(alcylène en $C_1$-$C_4$)-$OSO_3H$ lié à un chaînon de pont -S-, -SO- ou -$SO_2$-.

**3.** Solution solide, comprenant au moins deux composés de formule générale I donnée dans la revendication 1, dans laquelle R a en plus la signification d'un hydrogène, **caractérisée** en ce que au moins un de ces composés se trouve dans le réseau cristallin d'un des autres composés.

**4.** Procédé de préparation de dérivés du 9-oxo-1,9a,10-triaza-9-hydro-anthracène, de formule générale I donnée dans la revendication 1, **caractérisé** en ce que on fait réagir les dérivés de l'acide anthranilique de formule générale II

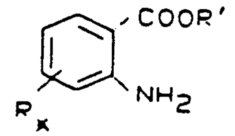

(II)

dans laquelle R' représente un atome d'hydrogène ou un groupe alkyle en C1-C4 ou aryle, R et x ont les significations indiquées dans la revendication 1, avec en premier lieu de 1 à environ 50 moles d'anhydride acétique, à une température comprise entre environ 50 et environ 180 °C, à une pression normale ou à une pression élevée ou réduite, ensuite avec de 1 à environ 10 moles d'hydrazine, à une

température comprise entre environ 0 et environ 120 °C, ensuite on fait réagir le produit obtenu de réaction de formule générale III

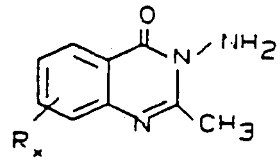

(III)

dans laquelle R et x ont les significations indiquées dans la revendication 1, avec de 1 à environ 2 moles, de la dicétone de formule générale IV

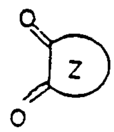

(IV)

dans laquelle Z a la signification indiquée dans la revendication 1, à une température comprise entre environ 140 et 240 °C, dans un solvant organique inerte au regard des réactifs, en éliminant dans le même temps l'eau se formant au cours de la réaction.

**5.** Procédé de préparation de dérivés du 9-oxo-1,9a,10-triaza-9-hydro-anthracène, de formule générale I donnée dans la revendication 1, **caractérisé** en ce que on fait réagir un dérivé de l'acide anthranilique de formule générale II

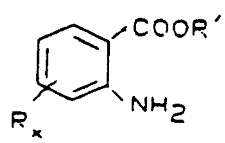

(II)

dans laquelle R' représente un atome d'hydrogène ou un groupe alkyle en $C_1$-$C_4$, un groupe phényle ou un groupe toluyle, R et x ont la signification donnée dans la revendication 2, dans un premier temps avec de environ 4 à environ 15 moles d'anhydride acétique à la température d'ébullition de l'anhydride acétique sous des conditions normales, ensuite avec de environ 2 à environ 6 moles d'hydrazine à une température comprise entre environ 15 et environ 70 °C et ensuite le produit de la réaction obtenu de formule générale III

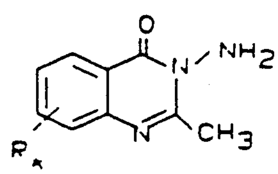

(III)

dans laquelle R et x ont les significations données dans la revendication 2, avec de environ 1,1 à environ 1,2 mole de 1,2-dicétone de formule générale IV

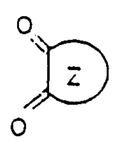

(IV)

dans laquelle Z a la signification donnée dans la revendication 2, ceci à une température comprise entre environ 160 et environ 180 ° C dans un solvant organique inerte vis-à-vis des réactifs avec dans le même temps élimination de l'eau formée au cours de la réaction.

**6.** Procédé selon au moins l'une des revendications 4 et 5, **caractérisé** en ce que la réaction avec la dicétone se fait dans l'o-dichlorobenzène, le diisopropylnaphtalène, le chloronaphtalène ou un mélange de 76,5 % d'éther diphénylique et 23,5 % de diphényle en tant que solvant inerte.

**7.** Procédé de préparation de la solution solide revendiqué dans la revendication 3, **caractérisé** en ce qu'on fait réagir au moins deux 3-amino-2-méthyl-4-oxoquinazoline différentes de formule générale III mentionnée dans la revendication 4 avec de 1 à environ 2 moles de 1,2-dicétone de formule générale IV mentionnée dans la revendication 4 par mole de 3-amino-2-méthyl-4-oxoquinazoline à une température comprise entre environ 140 et environ 240 ° C.

**8.** Procédé de préparation de la solution solide revendiquée dans la revendication 3, **caractérisé** en ce que on dissout au moins deux composés différents de formule générale I donnée dans la revendication 1 dans un acide inerte vis-à-vis des composés et on hydrolyse la solution obtenue en la versant dans de l'eau.

**9.** Procédé selon la revendication 8, **caractérisé** en ce qu'on dissout les composés de formule générale I dans une quantité pondérale d'environ 5 à environ 10 fois d'acide sulfurique de 96 à 100 %.

**10.** Utilisation des composés décrits dans au moins une des revendications 1, 2 et 3 pour la mise en couleur de matériaux naturels ou synthétiques.